# EUROPEAN PATENT APPLICATION

(11) **EP 3 146 907 A1**
(43) Date of publication of application: **29.03.2017**
(21) Application number: 15796497.4
(22) Date of filing: 28.04.2015
(51) Int. Cl.: A61B 17/04

(54) **THREAD AFFIXATION SYSTEM**

(30) Priority: 21.05.2014 JP 2014104892
(71) Applicant: Olympus Corporation, Hachioji-shi Tokyo 192-8507 (JP)
(72) Inventor: TAKAHASHI Shinji, Tokyo 192-8507 (JP)
(74) Representative: Schicker, Silvia
(86) International application number: PCT/JP2015/062900
(87) International publication number: WO 2015/178182

(57) **Abstract**

A suture-fixing system includes: an outer sheath which is insertable into a body; a suture-fixing tool having a first opening and a second opening configured to communicate with each other, the suture-fixing tool disposed at a distal end of the outer sheath; a lead-in tool which is inserted into the suture-fixing tool and is movable inside the outer sheath; and an engaging portion which is disposed at a distal end of the lead-in tool and extends in a loop shape or a U-shape from one of the first opening and the second opening which is located more distally than another; wherein both the suture-fixing tool and the engaging portion, or only the engaging portion is capable of forming an encircled area through which a portion of a surgical suture locked to a tissue can pass, using a medical instrument which sutures tissue with the surgical suture, and the engaging portion moves the portion of the surgical suture toward more proximal position than a proximal opening of the suture-fixing tool by the lead-in tool being moved to a more proximal position of the outer sheath.

## Description

### [Technical Field]

The present invention relates to a suture-fixing system, the suture-fixing system, and a lens assembly.

Priority is claimed on Japanese Patent Application No. 2014-104892, filed May 21, 2014, the content of which is incorporated herein by reference.

### [Background Art]

In the related art, a medical instrument and a method for suturing tissue in a body using a surgical suture are known. A method of ligating an operating surgical suture which maintains a sutured state of tissue by fixing an end of the surgical suture using a ring-shaped member is known (for example, refer to Patent Literature 1).

### [Citation List]

### [Patent Literature]

### [Patent Literature 1]

Japanese Unexamined Patent Application, First Publication No. H10-277044

### [Summary of Invention]

### [Technical Problem]

However, in the above related art, the end of the surgical suture is fixed while the surgical suture is pulled such that the sutured state does not loosen when the surgical suture is fixed. In this case, an operation of fixing the end of the surgical suture while pulling the surgical suture is complicated.

The present invention is made in view of the aforementioned circumstances, and an object of the present invention is to provide a suture-fixing system which is able to fix a surgical suture easily while the suturing of tissue being hard to be loosened.

### [Solution to Problem]

A suture-fixing system according to a first aspect of the present invention includes: an outer sheath which is insertable into a body; a suture-fixing tool having a first opening and a second opening configured to communicate with each other, the suture-fixing tool disposed at a distal end of the outer sheath; a lead-in tool which is inserted into the suture-fixing tool and is movable inside the outer sheath; and an engaging portion which is disposed at a distal end of the lead-in tool and extends in a loop shape or a U-shape from one of the first opening and the second opening which is located more distally than another; wherein both the suture-fixing tool and the engaging portion, or only the engaging portion is capable of forming an encircled area through which a portion of a surgical suture locked to tissue can pass, using a medical instrument which suture tissue with the surgical suture, and the engaging portion moves the portion of the surgical suture toward more proximal position than a proximal opening of the suture-fixing tool by the lead-in tool being moved to a more proximal position of the outer sheath.

According to a second aspect of the present invention, in the suture-fixing system according to the first aspect, the suture-fixing system may further include: a pusher which is disposed inside the outer sheath at a more proximal position than the suture-fixing tool, the pusher being configured to push a proximal end of the suture-fixing tool toward a distal position by being moved in a direction along a central line of the outer sheath.

According to a third aspect of the present invention, in the suture-fixing system according to the first aspect, the engaging portion may be bent outward in a radial direction of the outer sheath.

According to a fourth aspect of the present invention, in the suture-fixing system according to the first aspect, the encircled area may have a size capable of inserting a first delivery member of a pair of delivery members which are configured to deliver the surgical suture in a suturing instrument which locks the surgical suture to the tissue, and the surgical suture may be engaged with the engaging portion by passing the surgical suture through the encircled area in a process of delivering the surgical suture from the first delivery member to a second delivery member of the pair of delivery members.

According to a fifth aspect of the present invention, in the suture-fixing system according to the first aspect, the suture-fixing system may further include: a cylindrical cap which connects the outer sheath to a distal end of an insertion part of an endoscope.

According to a sixth aspect of the present invention, in the suture-fixing system according to the fifth aspect, the cap may include a holding portion which holds the engaging portion.

A suture-fixing system according to a seventh aspect of the present invention, includes: a cylindrical cap which is connectable to a distal end of an insertion part of an endoscope; a suture-fixing tool which has a first opening and a second opening configured to communicate with each other, the suture-fixing tool being disposed in the cap; and a lead-in tool which is inserted into the suture-fixing tool, the lead-in tool including an engaging portion configured to extend in a loop shape or a U-shape from one of the first opening and the second opening which is located more distally than another, the lead-in tool expanding through an inside of the cap from one of the first opening and the second opening which is located more proximally than another to a proximal side of the cap, wherein the engaging portion configured to be attached to the cap to follow a distal opening of the cap, the engaging portion having an encircled area configured to have a portion of a medical instrument protruding from the insertion part to suture tissue using a surgical suture pass, a portion of the surgical suture locked to the tissue is caused to pass through the encircled area by the medical instrument so that the surgical suture is engaged with the engaging portion, and the engaging portion causing a portion of the surgical suture locked to the tissue to move to a more proximal position than the more proximal opening of the suture-fixing tool by the lead-in tool being moved toward a proximal side of the cap while the surgical suture is engaged with the engaging portion.

### [Advantageous Effects of Invention]

According to the suture-fixing system of the aforementioned aspects, a surgical suture can easily be fixed while the suturing of tissue being hard to be loosened.

### [Brief Description of Drawings]

Fig. 1 is an overall diagram of a suture-fixing system of a first embodiment of the present invention.
Fig. 2 is a cross-sectional view of the suture-fixing system.
Fig. 3 is a cross-sectional view showing another constitution example of a suture-fixing tool in the suture-fixing system.
Fig. 4 is a partial cross-sectional view showing another constitution example of an engaging portion in the suture-fixing system.
Fig. 5 is a schematic diagram showing an example of a suturing instrument used with the suture-fixing system.
Fig. 6 is a schematic diagram showing an example of a medical instrument which can be used instead of the suturing instrument.
Fig. 7 is a view for describing an action of the suture-fixing system.
Fig. 8 is a view for describing an action of the suture-fixing system.
Fig. 9 is a view for describing an action of the suture-fixing system.
Fig. 10 is a view for describing an action of the suture-fixing system.
Fig. 11 is a view for describing an action of the suture-fixing system.
Fig. 12 is a view for describing an action of the suture-fixing system.
Fig. 13 is a view for describing an action of the suture-fixing system.
Fig. 14 is a view for describing an action of the suture-fixing system.
Fig. 15 is a view for describing an action of the suture-fixing system.
Fig. 16 is a view for describing an action of the suture-fixing system.
Fig. 17 is a view for describing an action of the suture-fixing system.
Fig. 18 is a view for describing an action of the suture-fixing system.
Fig. 19 is a cross-sectional view showing a constitution of a modified example of the suture-fixing system.
Fig. 20 is a view for describing an action of the suture-fixing system.
Fig. 21 is a partial cross-sectional view showing a portion of a suture-fixing system in a second embodiment of the present invention.
Fig. 22 is a cross-sectional view showing a suture-fixing tool of the suture-fixing system.
Fig. 23 is a view for describing an action of the suture-fixing system.
Fig. 24 is a view for describing an action of the suture-fixing system.
Fig. 25 is a view for describing an action of the suture-fixing system.
Fig. 26 is a view for describing an action of the suture-fixing system.
Fig. 27 is a view for describing an action of the suture-fixing system.
Fig. 28 is a view for describing an action of the suture-fixing system.
Fig. 29 is a view for describing an action of the suture-fixing system.
Fig. 30 is a view for describing an action of the suture-fixing system.
Fig. 31 is a view for describing an action of the suture-fixing system.
Fig. 32 is a cross-sectional view showing a constitution of a modified example of the suture-fixing system.
Fig. 33 is a view for describing an action of the suture-fixing system.
Fig. 34 is a cross-sectional view showing a constitution of another modified example of the suture-fixing system.
Fig. 35 is a cross-sectional view showing another constitution example in the modified example.

### [Description of Embodiments]

### (First embodiment)

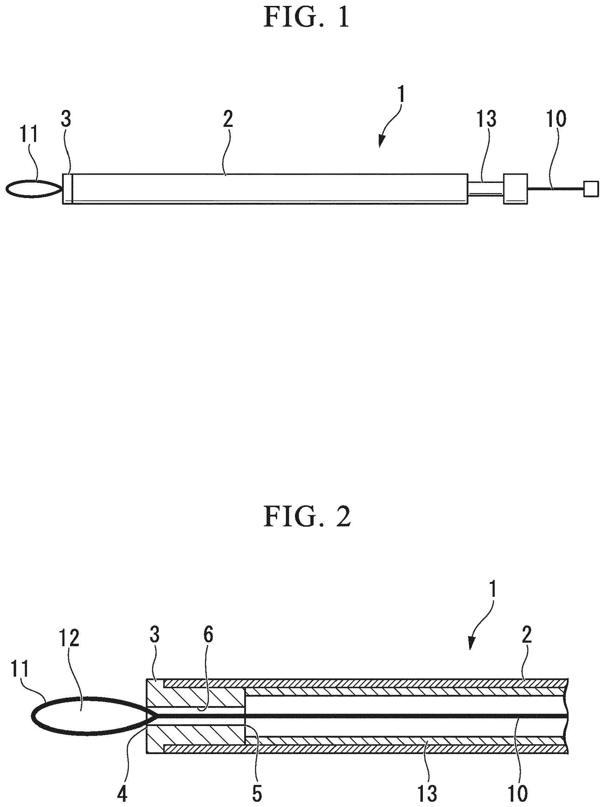

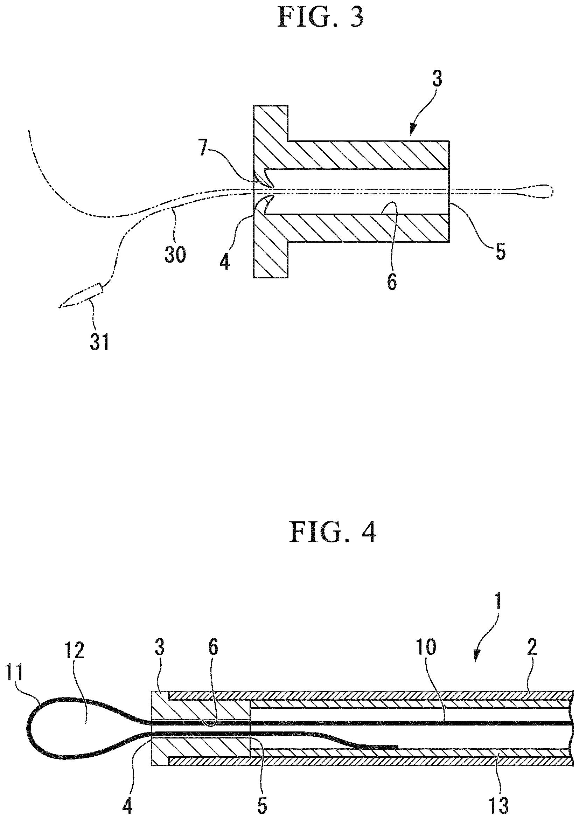

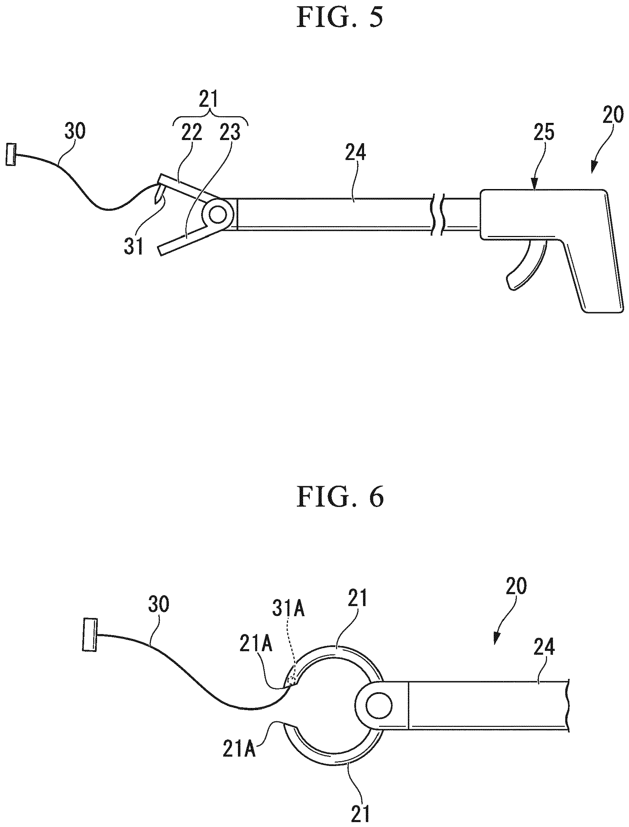

A first embodiment of the present invention will be described. Fig. 1 is an overall diagram of a suture-fixing system of a first embodiment of the present invention. Fig. 2 is a cross-sectional view of the suture-fixing system. Fig. 3 is a cross-sectional view showing another constitution example of a suture-fixing tool in the suture-fixing system. Fig. 4 is a partial cross-sectional view showing another constitution example of an engaging portion in the suture-fixing system. Fig. 5 is a schematic diagram showing an example of a suturing instrument used with the suture-fixing system. Fig. 6 is a schematic diagram showing an example of a medical instrument which can be used instead of the suturing instrument.

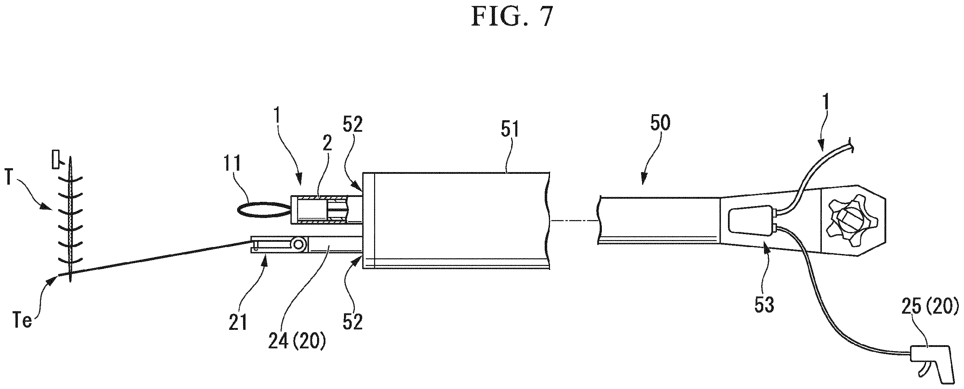

A suture-fixing system 1 of this embodiment shown in Fig. 1 is, for example, a medical system which can be used with an endoscope 50 shown in Fig. 7.

In this embodiment, the endoscope 50 used with the suture-fixing system 1 is, for example, a direct view type flexible endoscope including two endoscopic channels 52. Note that a constitution of the endoscope 50 is not particularly limited as long as the endoscope 50 has a constitution (for example, the endoscopic channels 52) in which an outer sheath 2 to be described below can be guided to a site to be treated.

The suture-fixing system 1 includes the outer sheath 2, a suture-fixing tool 3, a lead-in tool 10, an engaging portion 11, and a pusher 13.

The outer sheath 2 is a flexible cylindrical member which can be inserted into a body via one of the endoscopic channels 52 of the endoscope 50 shown in Fig. 7. A size of an outer diameter of the outer sheath 2 is a size in which the outer sheath 2 can be moved inside the endoscopic channel 52 in accordance with a constitution of the endoscopic channel 52.

As shown in Fig. 2, the suture-fixing tool 3 is disposed at a distal end portion of the outer sheath 2. The suture-fixing tool 3 is a cylindrical member having an outer diameter in which the suture-fixing tool 3 can be inserted into the outer sheath 2. A first opening 4 is formed at a distal portion of the suture-fixing tool 3. A second opening 5 is formed at a proximal portion of the suture-fixing tool 3. The first opening 4 and the second opening 5 communicate with each other through a passage 6 through which the lead-in tool 10 and the engaging portion 11 can pass.

An inner diameter of the passage 6 of the suture-fixing tool 3 is set to be big enough that a frictional force which enables a surgical suture 30, which will be described below, to be temporarily fixed to an inner surface of the passage 6 is generated. The inner diameter of the passage 6 of the suture-fixing tool 3 can be set in accordance with a constitution (for example, a material, a thickness, etc.) of the surgical suture 30. The suture-fixing tool 3 of this embodiment has a cylindrical shape. A material of the suture-fixing tool 3 is a material which can be plastically deformed so as to reduce the inner diameter of the passage 6. For example, the suture-fixing tool 3 is constituted by a metal having biocompatibility.

As another constitution in which the inner diameter of the passage 6 is reduced, the suture-fixing tool 3 may have a flap 7 formed on the inner surface of the passage 6 to enable the surgical suture 30 to be moved through the passage 6 in only one direction as shown in Fig. 3.

The lead-in tool 10 shown in Fig. 2 is a flexible linear member. The lead-in tool 10 is disposed inside the outer sheath 2 to be able to be moved inside the outer sheath 2. Also, the lead-in tool 10 is inserted into the passage 6 of the suture-fixing tool 3, and extends toward a more proximal position inside the outer sheath 2 than the suture-fixing tool 3 in a state in which the suture-fixing tool 3 is attached to the outer sheath 2. A proximal end of the lead-in tool 10 can be used as an operating portion which is used when a user of the suture-fixing system 1 advances and retracts the lead-in tool 10 with respect to the outer sheath 2. A material of the lead-in tool 10 may be appropriately selected from well-known flexible materials which can be molded in a linear shape. For example, a metal, a resin, etc. can be adopted as the material of the lead-in tool 10.

As shown in Fig. 2, the engaging portion 11 is disposed at a distal end of the lead-in tool 10. In this embodiment, the engaging portion 11 is a linear member which has an annular portion of a closed loop shape forming an encircled area 12 therein. The engaging portion 11 can be deformed to be able to pass through the passage 6 of the suture-fixing tool 3. The annular portion of the engaging portion 11 is restored, while an external force is not applied, such that the encircled area 12 with a size in which at least a portion of the surgical suture 30 can be inserted into the encircled area 12 is formed inside the engaging portion 11. A material of the engaging portion 11 may be appropriately selected from well-known flexible materials which can be molded in a linear shape. For example, a metal, a resin, etc. can be adopted as the material of the engaging portion 11.

The engaging portion 11 may be connected to the lead-in tool 10 using a method such as caulking and may be integrally molded with the lead-in tool 10.

A shape of the engaging portion 11 is not limited to the shape having the annular portion of the closed loop shape as long as the encircled area 12 with the size in which at least a portion of the surgical suture 30 can be inserted into the encircled area 12 can be formed. As shown in Fig. 4, for example, the engaging portion 11 may be formed such that the engaging portion 11 has a J shape as a whole and a portion which extends from the first opening 4 of a distal end in the suture-fixing tool 3 has a U-shape. In this case, the encircled area 12 through which the surgical suture 30 can pass is formed by the distal end of the suture-fixing tool 3 and the U-shape portion of the engaging portion 11.

As shown in Figs. 1 and 2, the pusher 13 is a cylindrical member. The lead-in tool 10 is inserted into the pusher 13 to be able to advance and retreat. The pusher 13 is movably inserted into the outer sheath 2 inside the outer sheath 2. A proximal end of the pusher 13 can be used as an operating portion used when the user of the suture-fixing system 1 advances and retracts the pusher 13 with respect to the outer sheath 2. The pusher 13 is disposed at a more proximal position than the suture-fixing tool 3 in a state in which the suture-fixing tool 3 is attached to the outer sheath 2. When the pusher 13 is moved toward a distal end of the outer sheath 2 along a central line of the outer sheath 2, the pusher 13 comes into contact with a proximal end surface of the suture-fixing tool 3. The pusher 13 can be further moved toward the distal end of the outer sheath 2 to push the suture-fixing tool 3 outside of the outer sheath 2 from the distal end of the outer sheath 2. A material of the pusher 13 may be appropriately selected from well-known materials which have flexibility and can be molded in a cylindrical shape. For example, a metal, a resin, etc. can be adopted as the material of the pusher 13.

Next, an example of a suturing instrument 20 serving as a medical instrument used with the suture-fixing system 1 of this embodiment will be described.

As shown in Fig. 5, the suturing instrument 20 is a medical instrument which sutures tissue using a surgical suture 30 with a suture needle 31 fixed to an end thereof. The suturing instrument 20 includes a pair of delivery members 21, a long member 24, and a hand operating portion 25.

The pair of delivery members 21 can be opened and closed.

The long member 24 can be inserted into one of the endoscopic channels 52 of the endoscope 50 to guide the pair of delivery members 21 to a site to be sutured T.

The hand operating portion 25 is disposed at a proximal end of the long member 24 to open and close the pair of delivery members 21.

In the suturing instrument 20 of this embodiment, a first delivery member 22 and a second delivery member 23 that make up the pair of delivery members 21 are opened and closed in accordance with an operation of the hand operating portion 25. The suturing instrument 20 can deliver the suture needle 31 from the first delivery member 22 to the second delivery member 23 or from the second delivery member 23 to the first delivery member 22 reversely in conjunction with the opening and closing operation. For this reason, in this embodiment, the surgical suture 30 can be locked to tissue to be sutured by passing the suture needle 31 through the tissue in the process of opening and closing the pair of delivery members 21.

In the suturing instrument 20, the pair of delivery members 21 may have puncture needle structures 21A which can puncture the tissue as shown in Fig. 6, instead of using the surgical suture 30 in which the suture needle 31 is fixed to an end thereof. In the suturing instrument 20, the puncture needle structures 21A may be caused to puncture the tissue while the puncture needle structures 21A hold a surgical suture 30 so that the surgical suture 30 passes through the tissue. In this case, the surgical suture 30 is delivered between the delivery members 21 in the pair of delivery members 21. In this case, a connecting tool 31A which can be connected to the pair of delivery members 21 may be attached to one end or both ends of the surgical suture 30.

In a process of fixing the suture-fixing tool 3 to the surgical suture 30 using the suture-fixing system 1, a delivery instrument including a pair of delivery members which have a function of delivering a surgical suture but do not have a function of passing the surgical suture through tissue may be used instead of the aforementioned suturing instrument 20.

Next, actions of the suture-fixing system 1 of this embodiment will be described. Figs. 7 to 18 are views for describing the actions of the suture-fixing system 1 of this embodiment.

In suturing tissue using the suture-fixing system 1, for example, as shown in Fig. 7, the endoscope 50 is guided to the site to be sutured T, and the suturing instrument 20 is guided to the site to be sutured T via one of the endoscopic channels 52 of the endoscope 50.

The suture-fixing system 1 is guided to a portion near the site to be sutured T via the endoscopic channel 52 different from the endoscopic channel 52 to which the suturing instrument 20 is attached in an insertion part 51 of the endoscope 50. At this time, the suture-fixing tool 3 is disposed inside the distal end of the outer sheath 2 of the suture-fixing system 1. The engaging portion 11 extends from the first opening 4 of the distal end of the suture-fixing tool 3. An amount of extension of the engaging portion 11 from the first opening 4 may be set to an extent at which suturing using the suturing instrument 20 is not obstructed.

When the suture-fixing system 1 is used, a wide operative field may be secured by housing the entire suture-fixing system 1 inside the endoscopic channel 52 as necessary.

When an extending direction of the engaging portion 11 from the first opening 4 is a direction in which the suturing using the suturing instrument 20 is obstructed, the entire suture-fixing system 1 may be rotated using a central line of the endoscopic channel 52 as a rotational center. In this case, the engaging portion 11 can be moved to a position at which the engaging portion 11 does not obstruct the suturing.

In the suturing using the suturing instrument 20, for example, as shown in Fig. 7, the sutured state is caused not to loosen in a suture site by pulling the surgical suture 30 after passing the surgical suture 30 through the tissue by several stitches from a suturing start portion Ts. For example, the endoscope 50 is caused to be moved together with the suturing instrument 20 in a direction in which the endoscope 50 is removed to the outside of the body to eliminate the loosening of the sutured state in the suture site.

Alternatively, when the suturing instrument 20 is caused to be moved with respect to the endoscopic channel 52 in a direction in which the suturing instrument 20 is removed from the endoscopic channel 52 of the endoscope 50, the loosening of the sutured state in the suture site can also be eliminated.

In the site sutured by the suturing instrument 20, the suture-fixing tool 3 is arranged on a suturing end portion and the suture-fixing tool 3 is fixed to the surgical suture 30 such that the suturing end portion does not escape from the tissue.

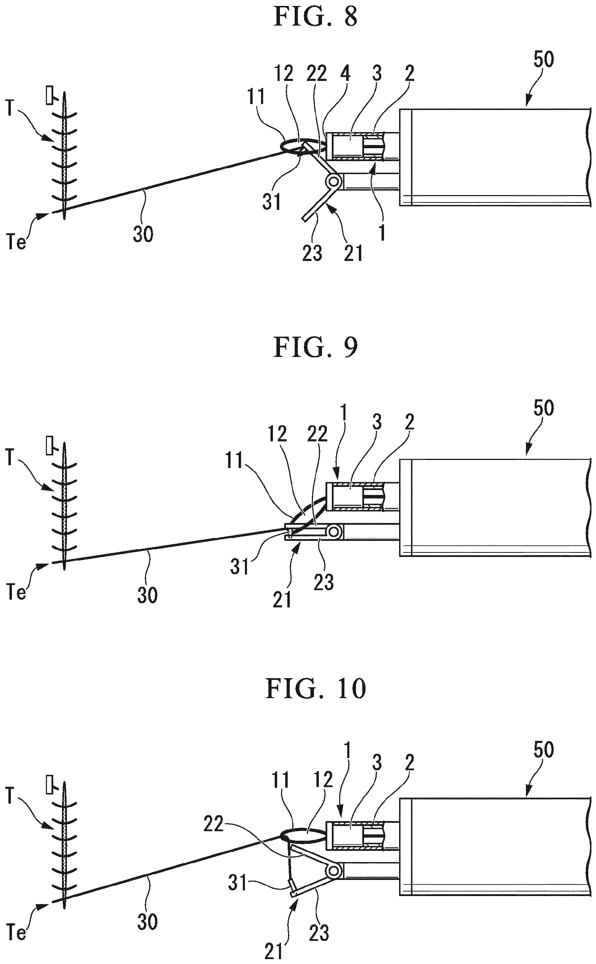

The surgical suture 30 is first caused to pass through the passage 6 of the suture-fixing tool 3 to fix the suture-fixing tool 3 to the surgical suture 30. To be specific, as shown in Fig. 8, a member (for example, the first delivery member 22) of the pair of delivery members 21 to which the suture needle 31 is connected is first inserted into the encircled area 12 formed by the engaging portion 11 extending from the first opening 4 of the distal side of the suture-fixing tool 3.

Subsequently, as shown in Figs. 9 and 10, the suture needle 31 is delivered from one of the pair of delivery members 21 to the other delivery member (for example, in this case, from the first delivery member 22 to the second delivery member 23) by closing the pair of delivery members 21 and then opening the pair of delivery members 21 again. The suture needle 31 passes through the encircled area 12 in the aforementioned opening and closing operation of the pair of delivery members 21. At this time, a portion of the surgical suture 30 also passes through the encircled area 12 together with the suture needle 31.

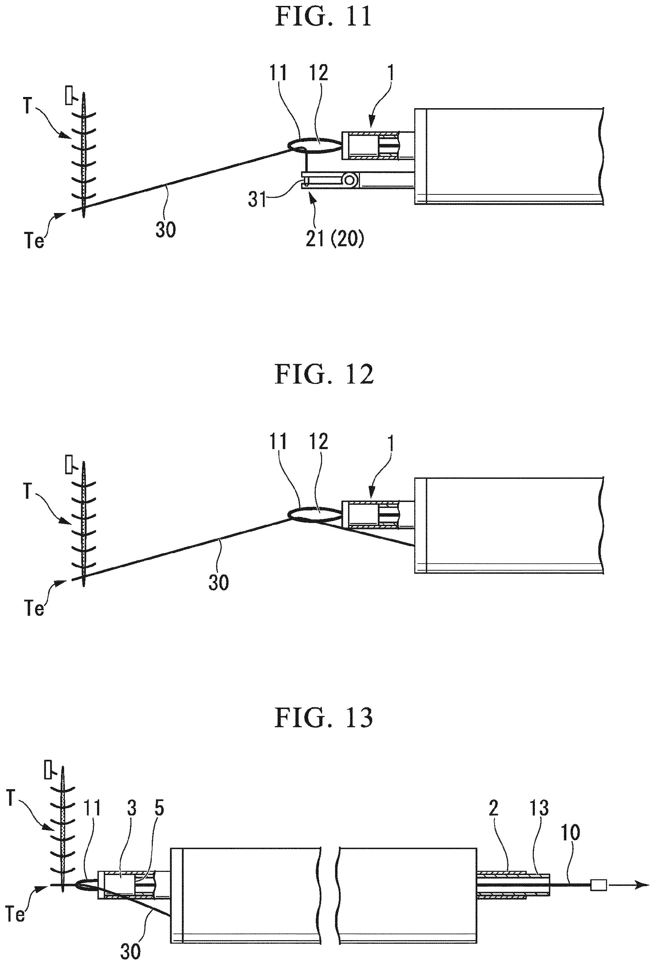

Subsequently, as shown in Figs. 11 and 12, the surgical suture 30 is pulled by drawing the pair of delivery members 21 into the endoscopic channel 52 such that the site to be sutured T is sutured by the surgical suture 30 without loosening.

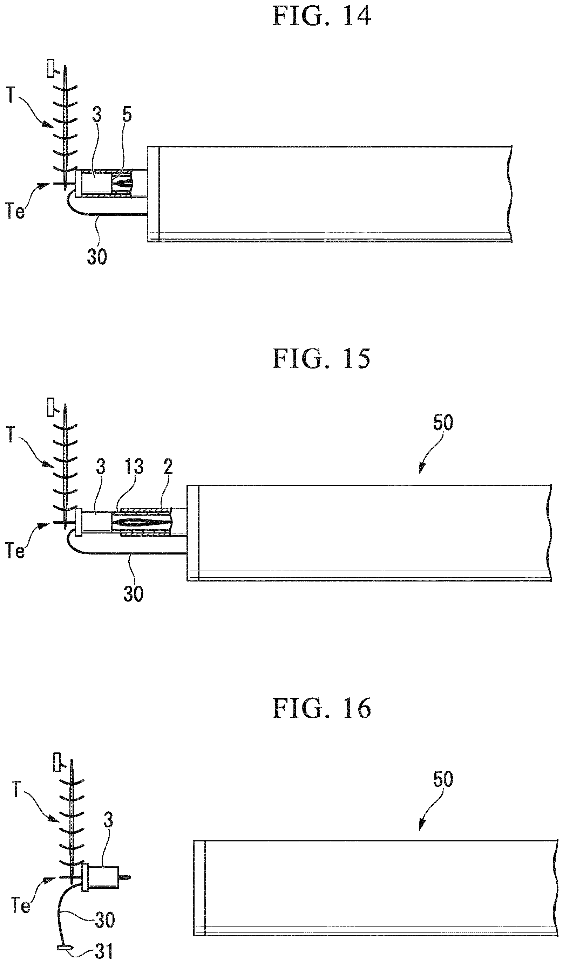

Subsequently, as shown in Figs. 13 and 14, the lead-in tool 10 is moved from the distal end of the outer sheath 2 toward the proximal end while the suture needle 31 is held by the pair of delivery members 21 through, for example, a manual operation of the user. When the lead-in tool 10 is moved by the user, the engaging portion 11 disposed at the distal end of the lead-in tool 10 is moved toward the proximal end together with the lead-in tool 10. The engaging portion 11 is moved from the first opening 4 of the distal side of the suture-fixing tool 3 toward the second opening 5 of the proximal side of the suture-fixing tool 3 via an inside of the passage 6. Since the surgical suture 30 is caused to pass through the encircled area 12 formed by the engaging portion 11, the surgical suture 30 is engaged with the engaging portion 11. The surgical suture 30 is moved from the first opening 4 of the distal side of the suture-fixing tool 3 to the second opening 5 of the proximal side of the suture-fixing tool 3 via the passage 6 together with the engaging portion 11.

Subsequently, the surgical suture 30 is pulled outside of the endoscopic device insertion port 53 (refer to Fig. 7) at the proximal end of the endoscopic channel 52 by further moving the engaging portion 11 toward the proximal end after the surgical suture 30 is caused to pass through the second opening 5 of the proximal side of the suture-fixing tool 3. When the surgical suture 30 is sufficiently long, the surgical suture 30 is pulled outside of the endoscope 50 from the endoscopic device insertion port 53 such that the suture needle 31 is not drawn into the endoscopic channel 52.

Subsequently, as shown in Fig. 15, the suture-fixing tool 3 is pushed outside of the outer sheath 2 from the distal end of the outer sheath 2 by moving the pusher 13 toward the distal side of the outer sheath 2. The pusher 13 can be moved through, for example, a manual operation of the user or the like.

The surgical suture 30 is locked to the passage 6 of the suture-fixing tool 3 by a frictional force at a temporarily fixing level. When the suture-fixing tool 3 is pushed by the pusher 13 to the distal side, the suture-fixing tool 3 is introduced and reaches a suturing end portion Te in the tissue along the surgical suture 30.

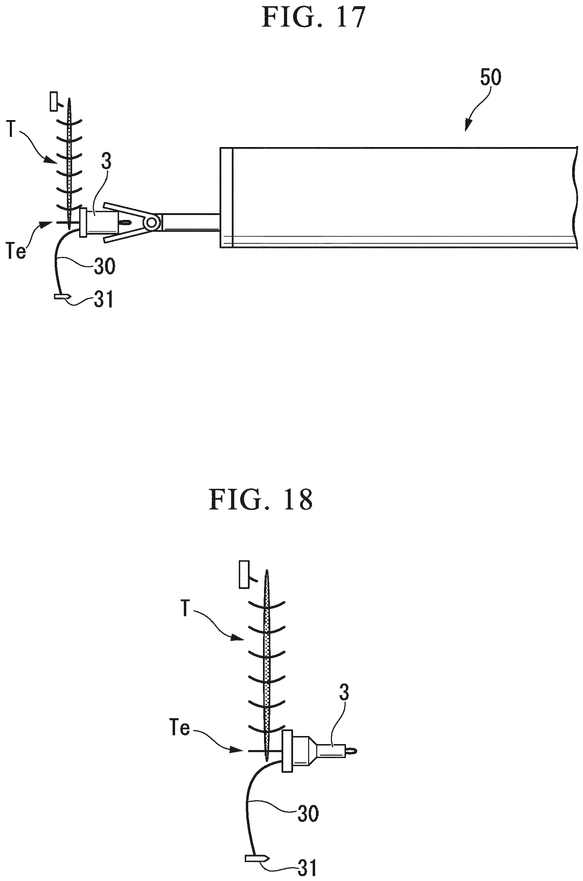

When the suture-fixing tool 3 reaches the suturing end portion Te in the tissue, redundant surgical suture 30 is cut by a scissors forceps or the like (not shown) and is taken outside of the body (refer to Fig. 16). Also, as shown in Fig. 17, the suture-fixing tool 3 is plastically deformed using a grasping forceps or the like such that the passage 6 (refer to Fig. 2) of the suture-fixing tool 3 is crushed.

For example, when the two endoscopic channels 52 are provided in the endoscope 50, the grasping forceps or the like, which plastically deforms the suture-fixing tool 3, may be attached to the endoscopic channel 52 of the two endoscopic channels 52 to which the suture-fixing system 1 is not attached. In this case, the suture-fixing tool 3 can be plastically deformed by the grasping forceps or the like attached to the endoscopic channel 52.

The grasping forceps or the like, which plastically deforms the suture-fixing tool 3, may be inserted into the endoscopic channel 52 to which the suture-fixing system 1 is attached when the grasping forceps or the like is used. In this case, the surgical suture 30 is left in the endoscopic channel 52, and the outer sheath 2, the lead-in tool 10, the engaging portion 11, and the pusher 13 are removed from the endoscopic channel 52 before the grasping forceps or the like is inserted. The grasping forceps or the like can plastically deform the suture-fixing tool 3 when the grasping forceps or the like is inserted into the endoscopic channel 52 in which only the surgical suture 30 remains and extending the grasping forceps or the like to the suture-fixing tool 3.

The inner surface of the passage 6 of the suture-fixing tool 3 comes into close contact with an outer surface of the surgical suture 30 when the suture-fixing tool 3 is plastically deformed, and the surgical suture 30 is thus fixed to the suture-fixing tool 3 as shown in Fig. 18.

As described above, according to the suture-fixing system 1 of this embodiment, the surgical suture 30 can be easily drawn into the suture-fixing tool 3 while the surgical suture 30 is stretched to eliminate the loosening of the surgical suture 30 after the surgical suture 30 is locked to the tissue to suture the tissue. Also, according to the suture-fixing system 1 of this embodiment, the suture-fixing tool 3 can be attached to the suturing end portion Te even while the surgical suture 30 is stretched to eliminate the loosening of the surgical suture 30.

As described above, according to the suture-fixing system 1 of this embodiment, the surgical suture 30 is easily fixed in the suturing of the tissue.

In addition, according to the suture-fixing system 1 of this embodiment, since the pusher 13 has a cylindrical shape, the suture-fixing tool 3 can be pushed to the distal side along the surgical suture 30 while the surgical suture 30 is inserted into the pusher 13. For this reason, according to the suture-fixing system 1 of this embodiment, the suture-fixing tool 3 can be easily moved to the suturing end portion Te.

### (Modified example)

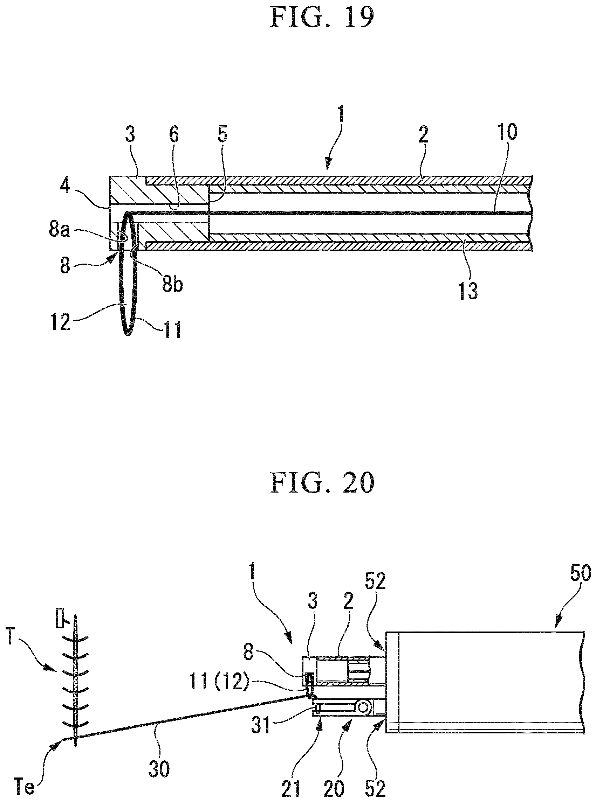

Next, a modified example of the aforementioned first embodiment will be described. Fig. 19 is a cross-sectional view showing a constitution of this modified example. Fig. 20 is a view for describing an action of a suture-fixing system of this modified example.

As shown in Figs. 19 and 20, in this modified example, a suture-fixing tool 3 includes a lateral opening 8 which communicates with the passage 6 and is opened in a lateral surface of the suture-fixing tool 3. The lateral opening 8 is formed in the lateral surface near the first opening 4 of the distal side between the first opening 4 and the second opening 5 described in the aforementioned first embodiment. The lateral opening 8 of this modified example is used instead of the first opening 4 of the aforementioned first embodiment.

The engaging portion 11 described in the aforementioned first embodiment can pass the surgical suture 30 through the suture-fixing tool 3 as in the aforementioned first embodiment by drawing the surgical suture 30 from the lateral opening 8 of the suture-fixing tool 3 to the passage 6 and further drawing the surgical suture 30 to the second opening 5 of the proximal side.

The lateral opening 8 of this modified example is formed as a slit having a shape in which a notch is formed in an outer wall portion of the suture-fixing tool 3 in a direction of a surface perpendicular to a central line of the passage 6 of the suture-fixing tool 3. The slit (the lateral opening 8) includes a wall surface 8a in a distal side and a wall surface 8b in a proximal side in a direction of the central line of the passage 6 of the suture-fixing tool 3.

If the engaging portion 11 is held in the slit (the lateral opening 8) in a predetermined posture, as described in the aforementioned first embodiment, the engaging portion 11 can be held in a direction in which the surgical suture 30 easily passes through the encircled area 12 when the surgical suture 30 is caused to pass through the encircled area 12 using the suturing instrument 20.

For example, the engaging portion 11 is held such that the engaging portion 11 comes into contact with the wall surface 8a of the distal side in the slit (the lateral opening 8). In this case, since the engaging portion 11 is bent in an L shape and protrudes to outside from the lateral surface of the suture-fixing tool 3, the surgical suture 30 can easily pass beside the suture-fixing tool 3.

For example, the engaging portion 11 is held such that the engaging portion 11 comes into contact with the wall surfaces 8a and 8b in the slit. The wall surfaces 8a and 8b extend at the distal side and the proximal side in the direction of the surface perpendicular to the central line of the passage 6 of the suture-fixing tool 3. In this case, the encircled area 12 formed by the engaging portion 11 is open in a circumferential direction of the suture-fixing tool 3 beside the lateral surface of the suture-fixing tool 3. For this reason, the surgical suture 30 can be moved in the circumferential direction beside the lateral surface of the suture-fixing tool 3 using the suturing instrument 20 to pass through the encircled area 12.

Also, the suture-fixing tool 3 is arranged on a suturing end portion Te as in the aforementioned embodiment after the surgical suture 30 is passed through the suture-fixing tool 3, and the suture-fixing tool 3 can thus be fixed to the surgical suture 30.

In the aforementioned embodiment, the engaging portion 11 advances and retracts in the direction of the central line of the endoscopic channel 52. On the other hand, in this modified example, since the engaging portion 11 is inserted into the lateral opening 8, the engaging portion 11 advances and retracts in a direction which is inclined with respect to the central line of the endoscopic channel 52. For this reason, for example, in the endoscope 50 in which the two endoscopic channels 52 are provided in parallel with each other, when the endoscope 50 is used while the suture-fixing system 1 is mounted on one of the two endoscopic channels 52 and the suturing instrument 20 is mounted on the other endoscopic channel 52, the engaging portion 11 can be moved toward the pair of delivery members 21 of the suturing instrument 20, for example, by rotating the outer sheath 2 using the central line of the endoscopic channel 52 as the rotational center. Therefore, in this modified example, the engaging portion 11 can be easily arranged at a position at which the pair of delivery members 21 of the suturing instrument 20 can easily pass through the encircled area 12.

### (Second embodiment)

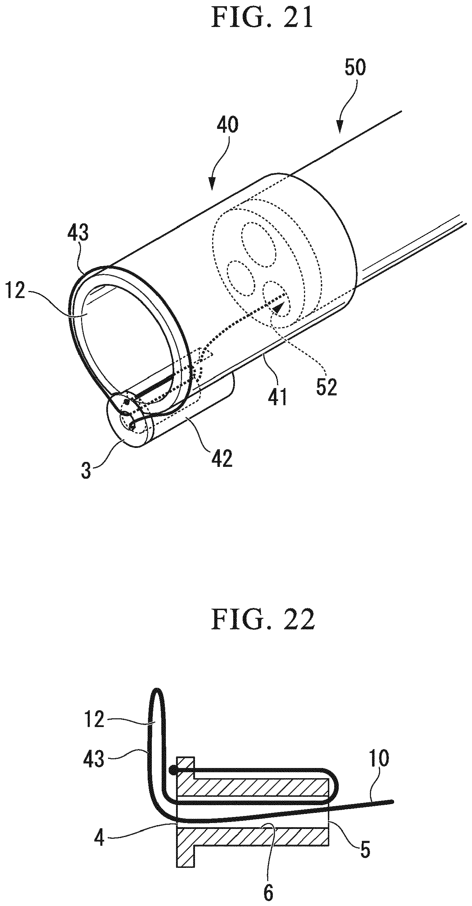

A second embodiment of the present invention will be described focusing on differences from the aforementioned first embodiment. Fig. 21 is a partial cross-sectional view showing a portion of a suture-fixing system in the second embodiment of the present invention. Fig. 22 is a cross-sectional view showing a suture-fixing tool of the suture-fixing system.

As shown in Figs. 21 and 22, in this embodiment, a cap 40 which can be attached to a distal end of an insertion part 51 in a well-known endoscope 50 is provided instead of the outer sheath 2 of the suture-fixing system 1 described in the aforementioned first embodiment.

This embodiment includes an engaging portion 43 fixed to a suture-fixing tool 3 instead of the engaging portion 11 described in the aforementioned first embodiment.

In this embodiment, the lead-in tool 10 described in the aforementioned first embodiment is inserted into one of endoscopic channels 52 of the endoscope 50 before a suture-fixing system 1 is used.

The cap 40 includes a cylindrical main body 41 to which a distal end of the insertion part 51 of the endoscope 50 can be inserted and a holding portion 42 which holds the suture-fixing tool 3 in the main body 41.

The main body 41 is, for example, transparent so that it does not obstruct an imaging field of vision of the endoscope 50.

A size and a shape of an opening of a distal side of the main body 41 are constituted such that usage of a medical instrument or the like which extends from the endoscopic channels 52 of the endoscope 50 is not obstructed.

The suture-fixing tool 3 described in the aforementioned first embodiment is inserted into the holding portion 42. The holding portion 42 locks an outer surface of the suture-fixing tool 3 by friction to hold the suture-fixing tool 3.

The engaging portion 43 is a flexible linear member. One end of the engaging portion 43 is fixed to the suture-fixing tool 3, and the other end thereof is connected to the lead-in tool 10. In this embodiment, the engaging portion 43 and the lead-in tool 10 are integrally molded with each other.

In other words, in this embodiment, the engaging portion 43 and the lead-in tool 10 are constituted by a series of linear members, one end of which is fixed to the suture-fixing tool 3, and the other end of which is pulled outside of the endoscope 50 via the endoscopic channel 52.

Fig. 22 shows a state in which the engaging portion 43 is attached to the suture-fixing tool 3.

The engaging portion 43 is inserted into a passage 6 of the suture-fixing tool 3 from the second opening 5 of a proximal side of the suture-fixing tool 3. The engaging portion 43 extends from the first opening 4 at the distal end of the suture-fixing tool 3 to a more distal position. Also, the engaging portion 43 is folded at a distal portion of the suture-fixing tool 3, and is inserted into the passage 6 of the suture-fixing tool 3 again from the first opening 4 at the distal end of the suture-fixing tool 3. The engaging portion 43 extends from the second opening 5 at the proximal end of the suture-fixing tool 3 to a more proximal position.

A portion which extends from the opening (the first opening 4) at the distal end of the suture-fixing tool 3 in the engaging portion 43 (hereinafter referred to as a distal side-extending portion of the engaging portion 43) forms an encircled area 12 that is big enough that the engaging portion 43 can be put around an outer circumference of the main body 41. The distal side-extending portion of the engaging portion 43 is attached to the main body 41. For this reason, in this embodiment, the engaging portion 43 of the portion in which the encircled area 12 is formed is arranged at a position at which the imaging field of vision is not obstructed by the endoscope 50.

As shown in Fig. 21, the distal side-extending portion of the engaging portion 43 forms the encircled area 12 that is big enough that the engaging portion 43 can be put around the outer circumference of the main body 41 and is attached to the main body 41. For this reason, the medical instrument, which extends from the endoscopic channels 52 when used, passes through the encircled area 12 without being caught by the distal side-extending portion.

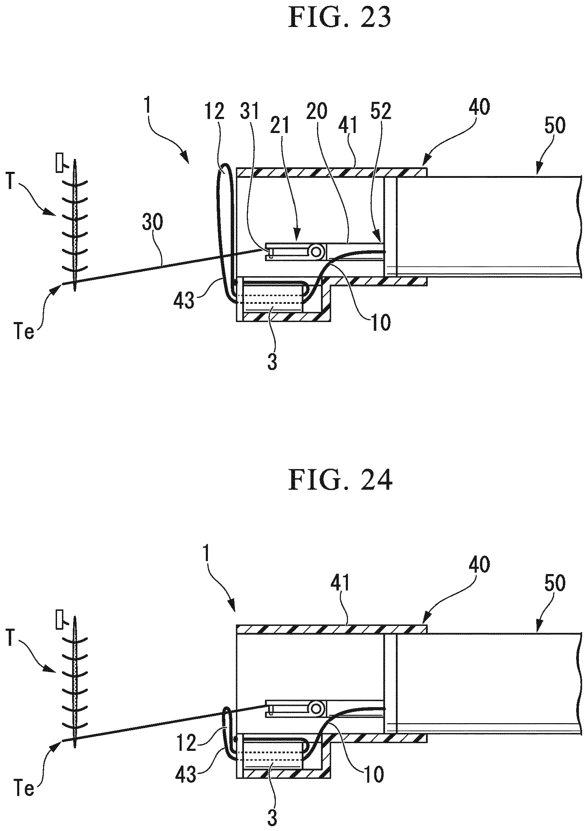

Actions of the suture-fixing system 1 of this embodiment will be described. Figs. 23 to 31 are views for describing the actions of the suture-fixing system 1 of this embodiment.

In this embodiment, for example, when the suturing instrument 20 described in the aforementioned first embodiment is attached to the endoscopic channel 52 of the endoscope 50, and a site to be sutured T is sutured, as shown in Fig. 23, the suturing instrument 20 passes through the encircled area 12 formed by the engaging portion 43 in a direction from a proximal side to a distal side and is guided to the site to be sutured T. The suturing instrument 20 passes through the encircled area 12 in a direction from the distal side to the proximal side in an opposite direction and returns after the surgical suture 30 is locked to tissue.

When the suturing instrument 20 is moved to the proximal side after the surgical suture 30 is locked to the tissue, a pair of delivery members 21 of the suturing instrument 20 are drawn into the endoscopic channel 52. At this time, since the surgical suture 30 is pulled by the suturing instrument 20, a suture site of the tissue is held without loosening.

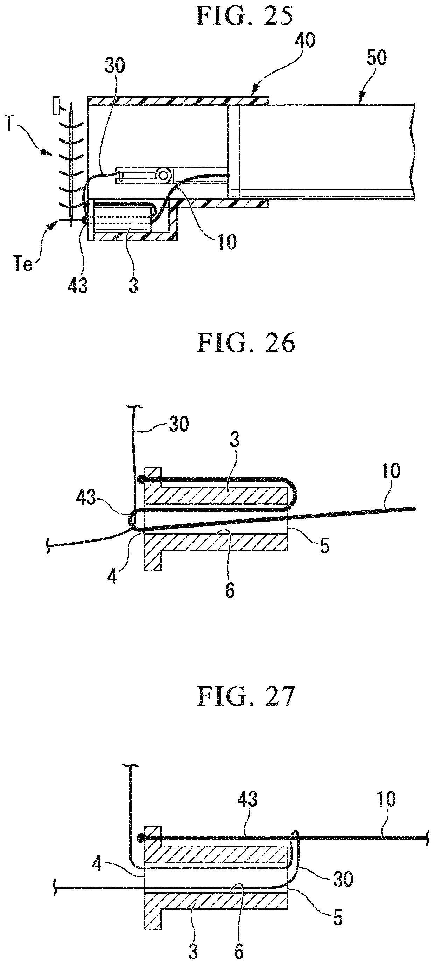

In this embodiment, when the surgical suture 30 is fixed using the suture-fixing tool 3, as shown in Figs. 24 and 25, a user of the suture-fixing system 1 (hereinafter simply referred to as the user) moves the insertion part 51 of the endoscope 50 such that the suture-fixing tool 3 is at a suturing end portion Te of the tissue. For example, the user moves the insertion part 51 of the endoscope 50 such that the first opening 4 at the distal end of the suture-fixing tool 3 comes into contact with the suturing end portion Te of the tissue.

When the suture-fixing tool 3 is arranged on the suturing end portion Te of the tissue while the surgical suture 30 is pulled by the suturing instrument 20, the suture-fixing tool 3 approaches the suturing end portion Te of the tissue while the surgical suture 30 does not loosen in the suture site.

The user pulls the lead-in tool 10 toward the proximal end of the endoscopic channel 52 in a state in which the suture-fixing tool 3 approaches the suturing end portion Te of the tissue after the surgical suture 30 passes through the encircled area 12 formed by the engaging portion 43 in the direction from the distal side to the proximal side.

As shown in Figs. 24 and 25, when the user pulls the lead-in tool 10 toward the proximal end of the endoscopic channel 52, the encircled area 12 formed by the engaging portion 43 is reduced.

Also, as shown in Figs. 26 and 27, the engaging portion 43, which forms the encircled area 12, is drawn from the first opening 4 of the distal end of the suture-fixing tool 3 into the passage 6. The engaging portion 43, which is drawn into the passage 6, moves the surgical suture 30 from the first opening 4 of the distal end of the suture-fixing tool 3 into the passage 6.

The lead-in tool 10 is moved toward the proximal end so that the engaging portion 43 passes through the passage 6. The engaging portion 43 is pulled outside from the second opening 5 of the proximal end of the suture-fixing tool 3 together with the surgical suture 30.

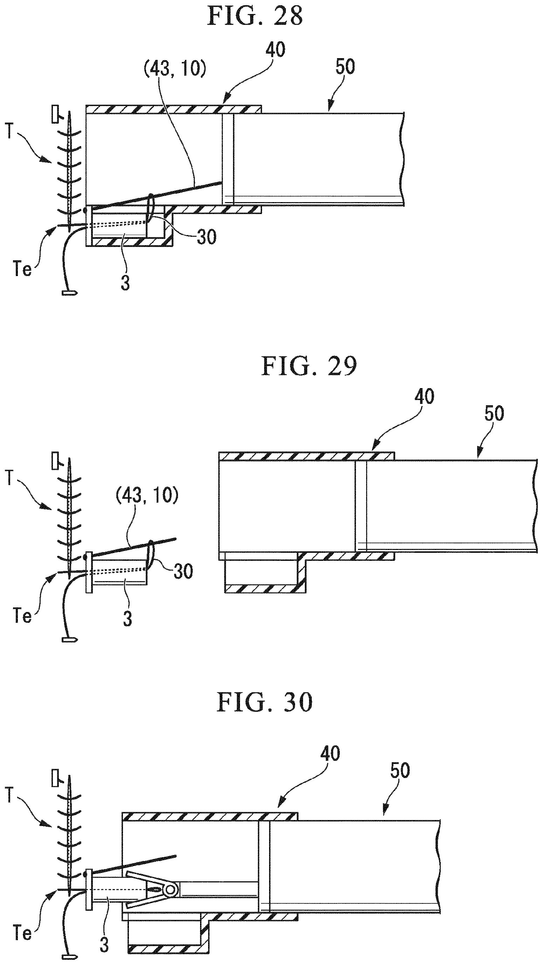

The surgical suture 30 is temporarily fixed to the suture-fixing tool 3 as in the aforementioned first embodiment in a state in which the surgical suture 30 is arranged inside the suture-fixing tool 3. As shown in Fig. 29, the user cuts the lead-in tool 10 or the engaging portion 43 in the endoscopic channel 52 as shown in Fig. 28 using the scissors forceps or the like (not shown).

When the lead-in tool 10 or the engaging portion 43 is cut, the suture-fixing tool 3 is moved outside of the cap 40 by moving the cap 40 and the endoscope 50 toward the proximal end. The suture-fixing tool 3 is placed on the suturing end portion Te of the tissue.

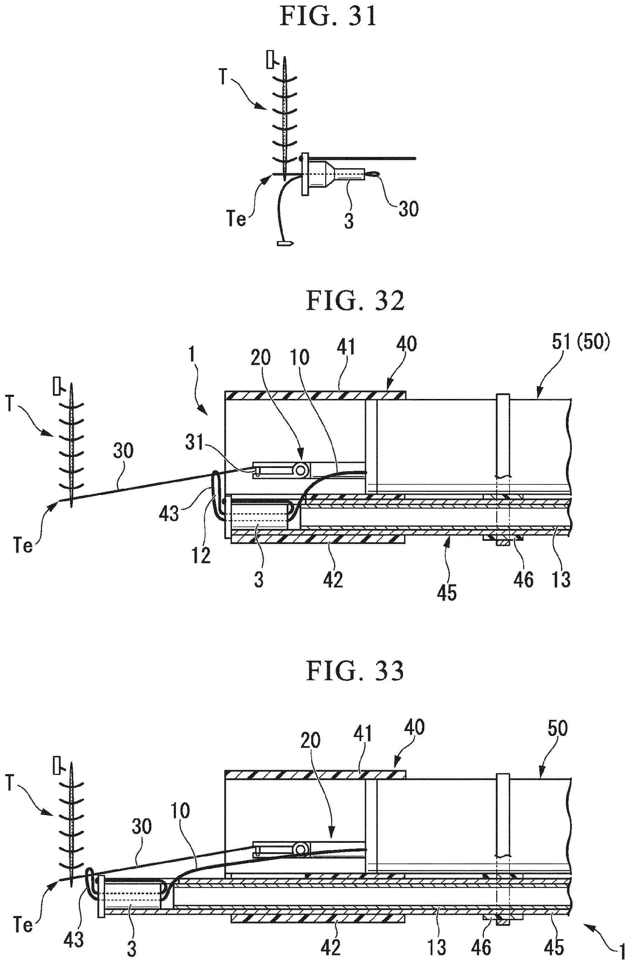

After that, the suture-fixing tool 3 is fixed to the surgical suture 30 as shown in Fig. 31 by plastically deforming the suture-fixing tool 3 using the grasping forceps or the like as shown in Fig. 30 as in the aforementioned first embodiment.

The suture-fixing system 1 of this embodiment accomplishes the same effect as the aforementioned first embodiment.

In the suture-fixing system 1 of this embodiment, the only member disposed in the endoscopic channel 52 is the lead-in tool 10. For this reason, even if the endoscopic channels 52 are too narrow for the suture-fixing system 1 described in the aforementioned first embodiment to be inserted, the suture-fixing tool 3 can be fixed to the surgical suture 30.

### (Modified example)

A modified example of the aforementioned second embodiment will be described. Fig. 32 is a cross-sectional view showing a constitution of a suture-fixing system of this modified example. Fig. 33 is a view for describing an action of the suture-fixing system.

As shown in Fig. 32, in this modified example, a holding portion 42 includes an external sheath 45. An external source 45 movably holds the pusher 13 described in the aforementioned first embodiment. It is noted that, in this modified example, the pusher 13 need not have a cylindrical shape.

The external sheath 45 is a flexible cylindrical member. The external sheath 45 can be attached to an outer surface of an insertion part 51 of an endoscope 50 via a cylindrical sleeve 46. For example, a binding band, a tape, etc. may be used to fix the external sheath 45 to the outer surface of the insertion part 51.

The external sheath 45 is movably inserted into the holding portion 42. A suture-fixing tool 3 is attached inside a distal end of the external sheath 45.

As shown in Figs. 32 and 33, the external sheath 45 can be moved in the sleeve 46 and the holding portion 42 toward a distal end of the endoscope 50 such that the suture-fixing tool 3 is arranged on a suturing end portion Te of tissue. For this reason, the external sheath 45 can move the external sheath 45 toward the distal end while the surgical suture 30 is pulled using the suturing instrument 20 in a state in which the surgical suture 30 passes through the encircled area 12. As described above, when the external sheath 45 is moved toward the distal end, the suture-fixing tool 3 attached to the distal end of the external sheath 45 is also guided to the suturing end portion Te of the tissue.

The pusher 13 is further used from this state as in the first embodiment so that the suture-fixing tool 3 is released from the external sheath 45.

In this modified example, the same effect as the first embodiment and the second embodiment is accomplished.

In this modified example, the suture-fixing tool 3 can be moved to the suturing end portion Te of the tissue more easily than in the second embodiment.

In this modified example, a force which brings the first opening 4 of the distal end of the suture-fixing tool 3 into contact with the suturing end portion Te of the tissue can be applied to the suture-fixing tool 3 through the external sheath 45. For this reason, in this modified example, it is more difficult for the surgical suture 30 to loosen than in the aforementioned second embodiment.

In this modified example, the pusher 13 disposed inside the external sheath 45 enables the suture-fixing tool 3 to be released from the external sheath 45 through a simple operation.

### (Modified example)

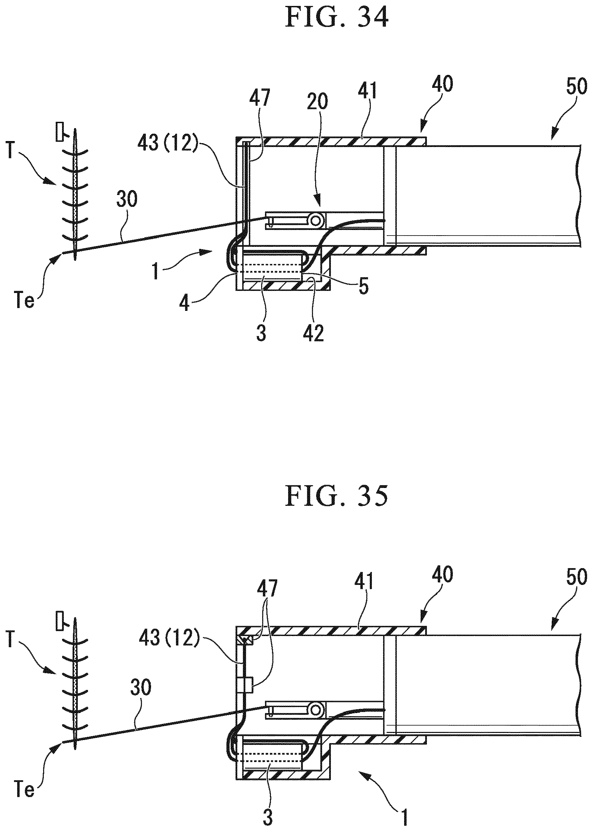

Another modified example of the aforementioned second embodiment will be described. Fig. 34 is a cross-sectional view showing a constitution of a suture-fixing system of this modified example. Fig. 35 is a cross-sectional view showing another constitution example in this modified example.

As shown in Fig. 34, in this modified example, a housing portion 47 which temporarily attaches an engaging portion 43 is provided inside a main body 41 of a cap 40.

The housing portion 47 includes, for example, a recessed shape which extends in a circumferential direction of the cap 40. The housing portion 47 can temporarily attach the annular engaging portion 43 along the recessed shape when the engaging portion 43 enters the cap 40 in a state in which the engaging portion 43 forms an encircled area 12 in an inner circumferential direction of the cap 40. As described above, the engaging portion 43 is held in the housing portion 47. At this time, the encircled area 12 with a shape along the inner circumferential surface of the cap 40 is formed inside the engaging portion 43 held in the housing portion 47.

In this modified example, for example, when the engaging portion 43 returns to the annular shape due to a restoring force of the engaging portion 43 itself to form the encircled area 12, the engaging portion 43 may be adapted to be locked to the housing portion 47 due to the restoring force of the engaging portion 43.

In this modified example, an inner surface of the housing portion 47 may be engaged with an outer surface of the engaging portion 43 which forms the encircled area 12 in a frictional manner so that the housing portion 47 holds the engaging portion 43.

In this modified example, the housing portion 47 may have a different constitution from that of the above description.

For example, as shown in Fig. 35, the housing portion 47 may be a hooking member which holds a portion of the engaging portion 43 which forms the encircled area 12 near an inner surface of the main body 41. The hooking member constituting the housing portion 47 has enough holding force that the engaging portion 43 is released by an amount of force by which the lead-in tool 10 pulls the engaging portion 43.

In this modified example, the encircled area 12 is formed by the engaging portion 43 arranged along the inner surface of the main body 41 inside the main body 41. For this reason, the surgical suture 30 is enabled to pass through the encircled area 12 using a medical instrument such as the suturing instrument 20 as in the aforementioned second embodiment.

In this modified example, it is more difficult for the engaging portion 43 to be caught on the main body 41 than when the encircled area 12 is formed by holding the engaging portion 43 outside the main body 41. For this reason, the encircled area 12 can be easily reduced.

Preferable embodiments of the present invention have been described above, but the present invention is not limited to these embodiments or the modified examples thereof. Addition, omission, replacement, and other changes of components are possible within a range not departing from the gist of the present invention.

The present invention is not limited to the foregoing description, but is only limited by the scope of the appended claims.

For example, in the aforementioned embodiment and the modified examples thereof, when the number of endoscopic channels 52 is insufficient in the endoscope 50 which is used with the suture-fixing system 1, the suture-fixing system 1 can be used by attaching an externally attached channel which is appropriately added to the endoscope 50.

### [Industrial Applicability]

According to the aforementioned embodiments (including the modified examples), a suture-fixing system which is able to fix a surgical suture easily while the suturing of tissue being hard to be loosened can be provided.

### [Reference Signs List]

- 1: Suture-fixing system
- 2: Outer sheath
- 3: Suture-fixing tool
- 4: First opening
- 5: Second opening
- 6: Passage
- 7: Flap
- 8: Lateral opening (slit)
- 8a: Wall surface of distal side of slit
- 8b: Wall surface of proximal side of slit
- 10: Lead-in tool
- 11: Engaging portion
- 12: Encircled area
- 13: Pusher
- 20: Suturing instrument
- 21: Pair of delivery members
- 21A: Puncture needle structure
- 22: First delivery member
- 23: Second delivery member
- 24: Long member
- 25: Hand operating portion
- 30: Surgical suture
- 31: Suture needle
- 31A: Connecting tool
- 40: Cap
- 41: Main body
- 42: Holding portion
- 43: Engaging portion
- 45: External sheath
- 46: Sleeve
- 47: Housing portion
- 50: Endoscope
- 51: Insertion part
- 52: Endoscopic channel
- 53: Endoscopic device insertion port

## Claims

1. A suture-fixing system, comprising:
an outer sheath which is insertable into a body;
a suture-fixing tool having a first opening and a second opening configured to communicate with each other, the suture-fixing tool disposed at a distal end of the outer sheath;
a lead-in tool which is inserted into the suture-fixing tool and is movable inside the outer sheath; and
an engaging portion which is disposed at a distal end of the lead-in tool and extends in a loop shape or a U-shape from one of the first opening and the second opening which is located more distally than another;
wherein both the suture-fixing tool and the engaging portion, or only the engaging portion is capable of forming an encircled area through which a portion of a surgical suture locked to tissue can pass, using a medical instrument which suture tissue with the surgical suture, and
the engaging portion moves the portion of the surgical suture toward more proximal position than a proximal opening of the suture-fixing tool by the lead-in tool being moved to a more proximal position of the outer sheath.

2. The suture-fixing system according to Claim 1, further comprising:
a pusher which is disposed inside the outer sheath at a more proximal position than the suture-fixing tool, the pusher being configured to push a proximal end of the suture-fixing tool toward a distal position by being moved in a direction along a central line of the outer sheath.

3. The suture-fixing system according to Claim 1, wherein the engaging portion is bent outward in a radial direction of the outer sheath.

4. The suture-fixing system according to Claim 1, wherein the encircled area has a size capable of inserting a first delivery member of a pair of delivery members which are configured to deliver the surgical suture in a suturing instrument which locks the surgical suture to the tissue, and
the surgical suture is engaged with the engaging portion by passing the surgical suture through the encircled area in a process of delivering the surgical suture from the first delivery member to a second delivery member of the pair of delivery members.

5. The suture-fixing system according to Claim 1, further comprising:
a cylindrical cap which connects the outer sheath to a distal end of an insertion part of an endoscope.

6. The suture-fixing system according to Claim 5, wherein the cap includes a holding portion which holds the engaging portion.

7. A suture-fixing system, comprising:
a cylindrical cap which is connectable to a distal end of an insertion part of an endoscope;
a suture-fixing tool which has a first opening and a second opening configured to communicate with each other, the suture-fixing tool being disposed in the cap; and
a lead-in tool which is inserted into the suture-fixing tool, the lead-in tool including an engaging portion configured to extend in a loop shape or a U-shape from one of the first opening and the second opening which is located more distally than another, the lead-in tool expanding through an inside of the cap from one of the first opening and the second opening which is located more proximally than another to a proximal side of the cap,
wherein the engaging portion configured to be attached to the cap to follow a distal opening of the cap, the engaging portion having an encircled area configured to have a portion of a medical instrument protruding from the insertion part to suture tissue using a surgical suture pass,
a portion of the surgical suture locked to the tissue is caused to pass through the encircled area by the medical instrument so that the surgical suture is engaged with the engaging portion, and
the engaging portion causing a portion of the surgical suture locked to the tissue to move to a more proximal position than the more proximal opening of the suture-fixing tool by the lead-in tool being moved toward a proximal side of the cap while the surgical suture is engaged with the engaging portion.
